# EUROPEAN PATENT SPECIFICATION

(11) **EP 3 628 219 B1**
(45) Date of publication and mention of the grant of the patent: **07.10.2026**
(21) Application number: 19209381.3
(22) Date of filing: 31.08.2012
(51) Int. Cl.: A61B 5/0215, A61B 5/00

(54) **PORTAL HYPERTENSION MONITORING SYSTEM**
PORTALES HYPERTONIE-MONITORING-SYSTEM
SYSTÈME DE SURVEILLANCE D'HYPERTENSION PORTALE

(30) Priority: 01.09.2011 US 201161530040 P
(43) Date of publication of application: 01.04.2020
(60) Divisional application: 26178017.5 / 4 778 451
(62) Document number(s) of the earlier application(s) in accordance with Art. 76 EPC: 12759322.6 / 2 750 587
(73) Proprietor: Microtech Medical Technologies Ltd., 6158101 Tel Aviv (IL)
(72) Inventor: RICHTER, Yoram, 47226 Ramat Hasharon (IL); TAMMAN, Eric, S., 71726 Modiin (IL); MANDEL, Shahar, Even-Dar, 69027 Tel Aviv (IL)
(74) Representative: Kuhnen & Wacker Patent- und Rechtsanwaltsbüro PartG mbB

(56) References cited:
- EP-A2- 1 068 836
- WO-A1-2009/132396
- WO-A2-2004/096007
- WO-A2-2005/024367
- WO-A2-2006/035275
- US-A1- 2005 049 499
- US-A1- 2007 220 986

## Description

### FIELD OF INVENTION

The present invention relates to a vibratable sensor for measuring fluid pressure in systems comprising incompressible fluids.

In particular, the present invention relates to a vibratable sensor for monitoring blood pressure, and the corresponding blood pressure gradient, between the portal and hepatic veins which together comprise the porto-hepatic venous system, via a small, passive, sensor that is deployed (implanted) in the portal vein only or in both the hepatic and portal veins. The sensor is capable of implantation in the porto-hepatic venous system due to its reduced dimensions, as compared to current sensors for measuring fluid pressure which are too large and invasive to allow frequent, accurate monitoring of porto-hepatic blood pressures. The implanted sensor measures portal vein blood pressure and/or the porto-hepatic venous pressure gradient by correlation between the blood pressure and the frequency response of the sensor, and may be used in a system which provides pressure readings via an external processing and display system.

### BACKGROUND

The portal vein is a vessel in the abdominal cavity that drains deoxygenated blood to the liver for cleaning. A system of blood vessels called the hepatic veins remove the cleaned blood from the liver to the inferior vena cava, where it is returned to the heart. Portal hypertension ("PHT") occurs when the portal vein experiences a rise in blood pressure that is not a consequence of an increase in a patient's overall systemic blood pressure. Often, PHT is defined according to a "portal pressure gradient," or, the difference in pressure between the portal vein and the hepatic veins, for example of 10 mmHg or greater. A typical portal venous pressure under normal physiological conditions is less than or equal to approximately 10 mmHg, and the hepatic venous pressure gradient (HVPG) is less than approximately 5 mmHg. Increased portal pressure leads to the formation of porto-systemic collaterals; the most serious of them being gastroesophageal varices. Once formed, varices represent a major risk for the patient due to the susceptibility for rupture and subsequent hemorrhage that in many cases leads to death. As a result, PHT is considered the most severe complication of cirrhosis of the liver and is the major cause of morbidity and mortality in cirrhosis patients.

Current procedures for monitoring portal pressure generally involve an indirect measurement of the portal venous pressure through the hepatic venous system. One such procedure is known as the hepatic venous pressure gradient or HVPG. HVPG is used to provide an indirect measurement of the portal vein pressure. The procedure is minimally invasive and involves catheterization of the hepatic venous system via femoral vein or jugular entry. A balloon tipped radiolucent catheter that is capable of measuring local blood pressure usually via a pressure transducer is placed in the Inferior Vena Cava or a large hepatic vein segment. Once in place the pressure is measured to provide the free hepatic venous pressure or FHVP. The FHVP is measured to quantify the external pressures being applied to the venous systems and to zero out the effects of systemic pressure. The catheter is then advanced into a small branch and a complete obstruction of flow is created (wedge position usually done by inflating balloon) to provide the wedged hepatic venous pressure or WHVP. The HVPG is given by HVPG = WHVP - FHVP. While the HVPG has been shown to be a very effective diagnostic and prognostic indicator, it has been limited by the invasiveness of the procedure and the need for standardization to provide reliable results.

Other indirect procedures include, for example, measurement of variceal pressure which employs esophago-gastric approaches to advance an inflatable balloon-catheter into the abdomen of patients via the esophagus and stomach and position the balloon, adjacent to a gastroesophageal varix. The force of inflation required against the wall of the varix is used to calculate the intravariceal blood pressure. In general, non-direct portal venous pressure measurement is less precise, while still invasive and uncomfortable for a patient.

Direct measurement of the portal vein has been attempted in the past. One such procedure involves puncture catheterization, wherein a radiologist accesses the portal and/or hepatic venous systems, under fluoroscopic guidance, by puncturing the tissue of the system with a needle or catheter from outside of the system. Using puncture catheterization, the portal vein may be accessed via a transhepatic puncture using either an intracostal or subxiphoid approach, wherein a needle or catheter is inserted at a patient's 12^{th} vertebrae, between the ribs, and punctures through to the portal vein. The hepatic venous system may be accessed via a transjugular approach, wherein a needle or catheter is inserted into the jugular vein and advanced into the hepatic vein via the vena cava. The portal vein may also be accessed from the hepatic venous system, using an intrahepatic puncture from the hepatic to portal venous systems. Thus, in order to monitor a portal pressure gradient, two separate punctures (for the portal and hepatic veins) are required. Physicians are reluctant to perform frequent, direct portal vein pressure measurements, due to the invasiveness of the procedure and as a result, it is not clinically practiced.

There exists a strong clinical need for a pressure monitoring system that can provide accurate pressure measurements of portal and/or hepatic blood pressure while allowing the physician to monitor those pressures non-invasively.

WO 2006/035275 to Shay Kaplan describes an implantable sensor which is protected from deposition of extraneous materials or tissue by a non-biological or biological barrier.

Conventional devices include active electronics, sensors, and controls which require a power supply, or a connection to the outside world, and which increase the size of conventional devices thus restricting their use in the porto-hepatic venous system. In addition, conventional devices rely on components, for example sensors and/or membranes, that are large and/or needed in plurality of sensors/membranes, in order to maintain functionality, due, in part, to their tendency to rupture.

A need therefore exists for a pressure measurement system that is small in size, sensitive in function, and does not require redundancy. In addition, a need exists for a sensor system that may be operated without the need for wires or cables to transmit the pressure experienced by the sensor to an external device. The pressure measurement system should be miniature, passive, implantable and wireless to allow for non-invasive, frequent monitoring of portal venous pressure.

### SUMMARY OF THE INVENTION

The present invention is defined in claim 1. Further improvements are subject to the dependent claims.

### BRIEF DESCRIPTION OF THE DRAWINGS

FIG. 1 shows a device for measuring portal venous pressure.
FIGS. 2, 2A and 2B show a sensor in accordance with the invention for measuring portal venous pressure.
FIG. 3 shows a system for measuring, interpreting, and displaying portal venous pressure.
FIG. 4 shows a passive sensor manufacturing method for the sensor .
FIGS. 5A-5C show various embodiments of a passive sensor anchoring device for the sensor.
FIGS. 6A-6B show aspects of various embodiments of a passive sensor implantation device for the sensor.
FIG. 7 illustrates exemplary resonance frequencies from a vibration sensor as a function of ambient pressure in response to three different excitation frequencies, based on pressure oscillations around the mean value to be measured.

### DETAILED DESCRIPTION OF THE INVENTION

The sensor of the invention generally relates to measuring ambient pressure in a system comprising an incompressible fluid. For purposes of this application, "incompressible fluid" refers generally to non-vapor, non-compressible, flowable media, such as liquids, slurries and gels. In particular, the sensor is implanted in a body to monitor hepatic and/or portal venous pressure. The miniature size of the apparatus, compared to current conventional devices for measuring ambient fluid pressure, and relatively low invasiveness of the sensor are particularly well suited to medical and physiological applications, including, but not limited to, measuring: i) blood vessel/artery/vein pressures such as, for example, in portal hypertension; ii) spinal fluid pressure in brain ventricles; iii) intra-abdominal pressures such as in the urinary tract, bladder, kidney, and bile ducts; and the like. The method may be applicable to any disease or condition involving bodily systems through which fluids, *i.e.,* incompressible fluids, e.g., liquids, flow.

The invention is discussed and explained below with reference to the accompanying drawings. The drawings are provided as an exemplary understanding of the invention and to schematically illustrate particular embodiments and details of the invention. The skilled artisan will readily recognize other similar examples equally within the scope of the invention. The drawings are not intended to limit the scope of the invention as defined in the appended claims.

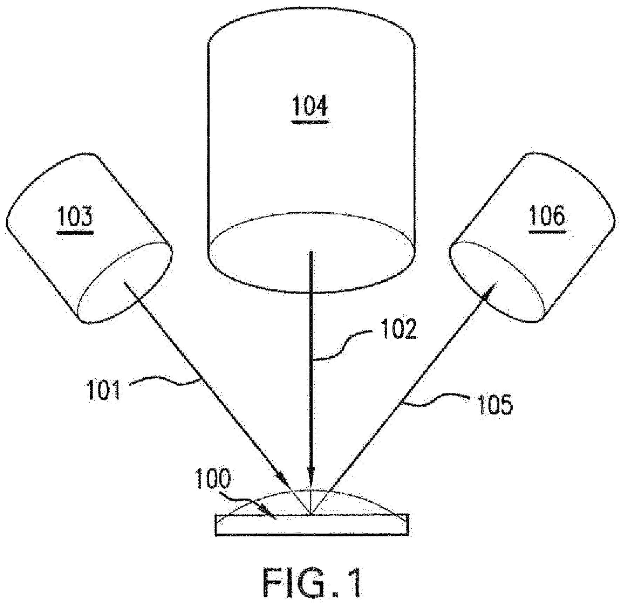

**Fig. 1** illustrates a sensor device system of the invention. Sensor device **100** measures ambient pressure of the implanted sensor device. Sensor device **100** is subjected to high frequency acoustic waves **101** and low frequency acoustic waves **102** which are generated by high frequency transmitter **103** and low frequency transmitter **104,** respectively. High frequency transmitter **103** and low frequency transmitter **104** may comprise any transducer suitable for controllably generating acoustic energy beams (such as, but not limited to sonic or ultrasonic beams) as is known in the art. Typically such transducers are called tactile transducers and are capable of converting an electrical signal into, for example, vibrations that may be felt or used for work. The transducers provide a field of view comprising a depth of penetration of 4-16 cm and a beam spot diameter of 3 cm generating a measurement ellipsoid, for example. The transducers may be implemented using suitable piezoelectric transducers, but other transducers known in the art may be used, such as, but not limited to, capacitive transducers, wideband capacitive transducers, composite piezoelectric transducers, electromagnetic transducers, various transducer array types and various suitable combinations of such transducers configured for obtaining different frequencies and/or beam shapes. For example, acoustic transmitters manufactured by Vemco, PCB Piezoelectronics, and Hardy Instruments may be used. Acoustic waves **101, 102** are directed at the sensor device **100,** producing modulated acoustic waves **105** that are detected by high frequency receiver **106.** Subsequent processing of waves **105** enables calculation of the ambient pressure in device **100.**

Disclosed herein is an implantable sensor device comprising a miniature sensor device for measuring ambient fluid pressure. The sensor device comprises a vibratable sensor in accordance with the present invention having a sensor membrane, which has a frequency response to ambient pressure conditions. The sensor membrane of the vibratable sensor forms one side of a chamber wherein resides a compressible gas of predefined pressure. The chamber is further defined by at least one wall which is preferably substantially perpendicular to the sensor membrane. In one embodiment, the vibratable sensor is made of silicon, but other suitable materials may be used, for example a metal, Pyrex^{®} or other glass, boron nitride, or the like. Non-limiting examples of metals include, *e.g.,* Titanium, Gold, Stainless Steel, Platinum, Tantalum, or any suitable metal, alloy, shape memory alloy such as NITINOL ^{®}. The chamber is sealed with a bonding layer forming a side of the chamber opposite the sensor membrane. The bonding layer for sealing the chamber, the bonding layer may also be used for attachment to an anchoring means. In one embodiment, the bonding layer provides a hermetic seal for the chamber disposed in the vibratable sensor. The bonding layer may comprise Pyrex^{®}, glass, silicon, or other suitable materials.

Generally, the vibratable sensor is manufactured by etching the appropriate shape and materials from a larger panel of the material. For example, the larger panel of material may be covered with a mask, the mask defining the shape of a plurality of the desired vibratable sensors, and then subjected to etching, which may be, for example, chemical etching or physical etching. The mask protects those areas of the panel that must not be removed during the etching process in order to produce the desired shape. For example, a plurality of vibratable sensors is formed when a mask having a plurality of precisely measured cut-outs cover a larger panel of material during the etching process, until chambers of the desired shape are produced in the larger panel to a depth that is substantially equal to a cut-out in the mask. The depth of the chamber may be controlled by various factors, for example where chemical etching is used: the volatility, duration, and number of chemical treatments. Each vibratable sensor may then be cut from the larger panel by slicing between consecutive chambers such that the amount of material remaining on each side of the chamber will be the thickness of walls defining a chamber in the vibratable sensor. The amount of material remaining between the bottom surface of the chamber and bottom of the larger panel will be the thickness of the sensor membrane. Any material that requires joining may be connected, for example, by brazing or welding.

As noted above, the vibratable sensor includes a bonding layer of, for example, Pyrex^{®} or other suitable material, in order to hermetically seal the vibratable sensor, preferably by joining the bonding layer to the walls of the chamber such that the bonding layer and sensor membrane are substantially parallel. In one embodiment, the bonding layer and sensor membrane form opposite walls of a vibratable sensor chamber. The bonding layer may provide a surface for attachment to anchors or other components.

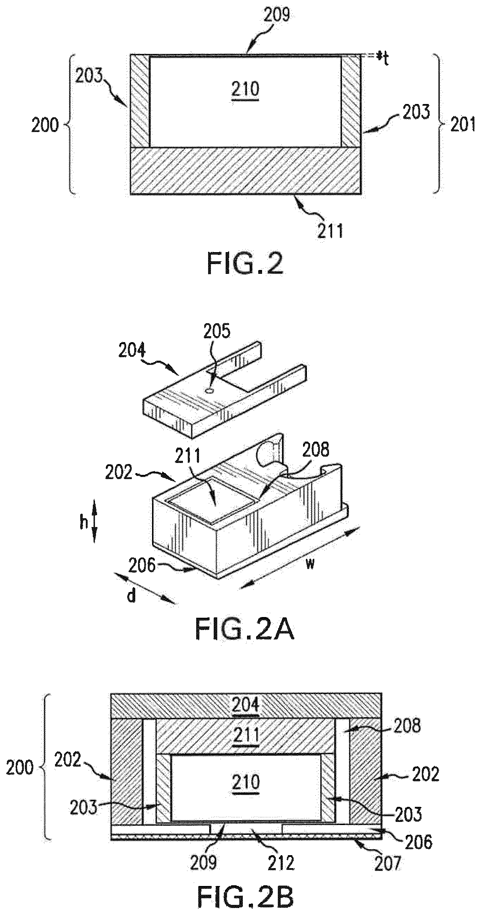

**Fig. 2** shows a cross sectional illustration of one embodiment of the sensor device **200.** In this embodiment, the sensor device **200** is a substantially cubic vibratable sensor **201.** As such, the sensor device **200** of **Fig. 2** comprises a sensor membrane **209** and chamber **210** which is sealed by bonding layer **211,** as described above. The sensor membrane **209** is comparatively thick relative to other remotely operated vibratable fluid pressure sensors. The sensor membrane **209** has a thickness in the range of 1 micron - 200 microns. Some exemplary but non-limiting thicknesses include 1.5 microns, 2 microns, 2.5 microns, and 5 microns. The sensor device **200** of the invention retains its accuracy despite the comparatively thick sensor membrane **209.** The use of a single sensor (as compared to the plurality of sensors required by prior art remotely-operated vibratable sensors) reduces the overall size of sensor device **200** compared to such conventional devices, making sensor device **200** suitable for use in the porto-hepatic venous system.

The vibratable sensor **201** has a height *h,* width *w*, and depth *d* In one embodiment, the vibratable sensor **201** measures 0.3 mm (*h*) x 0.5 mm (*w*) x 0.5 mm (*d*)*.* The width and depth of the vibratable sensor may be equal resulting in a substantially cubic structure. However, the dimensions of the vibratable sensor **201** are any dimensions that do not exceed a maximum volume of about 0.3 mm³, preferably having a size of equal to or less than 0.125 mm³. A minimum volume for the vibratable sensor **201** is about 0.008 mm³. Various alternative embodiments of the vibratable sensor **201** have volumetric ranges (in mm³) of, *e.g.,* 0.005-0.008, 0.01-0.09, or 0.1-0.3, as use requires. Vibratable sensor **201** a hermetically sealed, substantially non-solid component, of any shape, which includes sensor membrane **209** and chamber **210,** in the example illustrated by **Fig. 2****.** Sensor membrane **209** is a side of the chamber **210** of the vibratable sensor **201.** The depth of the chamber **210** is defined by the height (*h*) of the walls **203** of the vibratable sensor **201.** The sensor membrane **209** may have a thickness (*t*) on the order of about 2 microns in thickness (*t*), but more generally, the thickness (*t*) of the sensor membrane **209** is greater than one micron and less than or equal to **200** microns. Thickness (*t*) is measured along the height dimension (*h*) as depicted in **Fig. 2****.**

Vibratable sensor **201** may comprise the cropped rectangular overall shape illustrated in **Fig. 2****,** or one or more other suitable shapes, including but not limited to a sphere, pyramid, trapezoid, or other symmetrical or non-symmetrical shape. In one embodiment, the vibratable sensor **201** comprises silicon. In another embodiment vibratable sensor **201** comprises titanium or another acoustically active material. In other embodiments, vibratable sensor **201** comprises a rubber, polymer, and/or a ceramic material. Alternatively, the vibratable sensor **201** may comprise any suitable material capable of being excited by acoustic stimulation. As used in this application, "silicon" refers to silica and silicates, glasses, cements, and ceramics; it also refers to the class of silicones for which it is a constituent element, including various synthetic plastic and rubber substances made of silicon, oxygen, carbon and hydrogen, for example.

In other embodiments of the sensor device **200,** illustrated in **Figs. 2A** and **2B****,** the vibratable sensor is disposed in a cavity **208** defined by a housing **202.** The housing **202** encloses the sides of the vibratable sensor **201** but not all or part of the sensor membrane **(209** in **Fig. 2** and **2B****,** unnumbered in **Fig. 2A**), and the bonding layer **211** faces a cover plate **204** which is mechanically fixed to one side of the housing and serves as a surface for attachment to an anchoring means in certain embodiments. In one aspect of the embodiment illustrated in **Fig. 2A****,** the cover plate **204** may include a fill port **205.** The fill port **205** may be used to fill the cavity **208** with an incompressible fluid. As illustrated in **Figs. 2A** and **2B****,** the housing **202** is disposed atop a base plate **206,** which provides a foundation for the housing **202** and holds the vibratable sensor **201** inside the cavity **208.** The base plate **206** may contain an orifice **212,** as shown in **Fig. 2B****,** which exposes the sensor membrane **209** to acoustic activity thereby allowing vibrations to reach and return from vibratable sensor **201.**

In the particular embodiment illustrated in cross-sectional view in **Fig. 2B** the vibratable sensor **201** is disposed in cavity **208** of housing **202,** wherein the orifice **212** in base plate **206** exposes all or a portion of the sensor membrane **209** of vibratable sensor **201** to an acoustically transparent bottom film **207.** Bottom film **207** is designed to allow for the transmission of acoustic waves, hydrostatic and hydrodynamic pressures from the surrounding environment. Depending upon the choice of material used for the bottom film **207,** it may also function to protect the sensor. When the bottom film **207** comprises a semi permeable material, the film protects the vibratable sensor from direct exposure to bodily tissues or other solid bodily matter. When the bottom film **207** comprises an impermeable material, the film **207** may completely protect the vibratable sensor from all bodily fluids and/or materials. In an embodiment wherein the bottom film **207** is impermeable to all fluids and solids, a fill port (not shown in **Fig. 2B**) may be used to fill the cavity **208** with an incompressible fluid. Bottom film **207** comprises any suitable bioinert material or combinations thereof, including but not limited to, titanium, gold, stainless steel, platinum, tantalum, or any suitable metal, alloy, shape memory alloy such as NITINOL ^{®}, silicon, glass, quartz, a ceramic material, a composite material, a metallic or non-metallic nitride, boron nitride, a carbide, a metal oxide, a non-metallic oxide, a polymer based material, a gel, and combinations thereof. Alternatively, bottom film **207** may comprise titanium in one embodiment, for example diffusion-bonded Grade I titanium. In various embodiments, bottom film **207** may substantially seal vibratable sensor **201** in cavity **208,** for example when bottom film **207** comprises a substantially non-porous material, or bottom film **207** may be porous, to varying degrees, and expose vibratable sensor **201** to bodily fluids and/or tissues. In the embodiment shown in **Fig. 2A****,** described above, bottom film **207** is absent from base plate **206.** In such an embodiment, the vibratable sensor **201** would be completely exposed to the ambient environment via orifice **212.**

Cover plate **204,** housing **202,** and base plate **206** may each comprise any suitable bioinert materials or combinations thereof, including but not limited to titanium, gold, stainless steel, platinum, tantalum, or any suitable metal, alloy, shape memory alloy such as NITINOL ^{®}, silicon, glass, quartz, a ceramic material, a composite material, a metallic or non-metallic nitride, boron nitride, a carbide, a metal oxide, a non-metallic oxide, a polymer based material, a gel, and combinations thereof. Alternatively, base plate **206** may comprise a Pyrex ^{®} material. Base plate **206,** housing **202,** and cover plate **204** comprise titanium in one embodiment, for example Grade I titanium. These components may be formed and assembled from separate pieces or may be formed as one element or combined elements to function as described above.

In the embodiment depicted by **Fig. 2B****,** the vibratable sensor **201** contained in the housing cavity **208** may be surrounded by bodily fluid, *e.g.*, blood-flow, which enters the cavity **208** via a porous or absent bottom film **207.** Alternatively, the vibratable sensor **201** may be surrounded by an incompressible fluid that is sealed in cavity **208** by a substantially solid or impermeable bottom film **207,** after the incompressible fluid is introduced to cavity **208** through fill port **205.** A substantially solid bottom film **207** also prevents the introduction of bodily fluids and/or tissues into cavity **208.**

Base plate **206** is relatively thin (in the *h* direction), generally, compared to the overall height of the device as shown in **Figs. 2A, 2B****.** In one embodiment, base plate **206** represents, for example, 100 microns of an approximately 500 micron overall device height. In other embodiments, base plate **206** may be 5%-20% of the overall device height, but is generally less than or equal to 40% of the overall device height. The height of the base plate **206** should generally be minimized to allow for a maximum cavity **208** volume, which contributes to the accuracy of the device and therefore an overall reduced size when compared to conventional, vibratable sensors having a housing. The base plate **206** also provides a foundation for the device assembly, and absorbs mechanical stresses by providing a sink material (a material to absorb force or energy) where such stresses may dissipate.

Bottom film **207** may be bonded to all or a portion of the base plate **206** and provides further tolerance for stresses. The relatively thin bottom film is generally on the order of 1-10 microns. In one embodiment, the bottom film **207** is desirably 4 microns in thickness. The thin bottom film **207** is generally more pliable than thicker components of the device and may absorb stresses from, for example, expansion and contraction due to changing temperatures. Bottom film **207** is designed to allow for the transmission of acoustic waves, hydrostatic and hydrodynamic pressures from the surrounding environment.

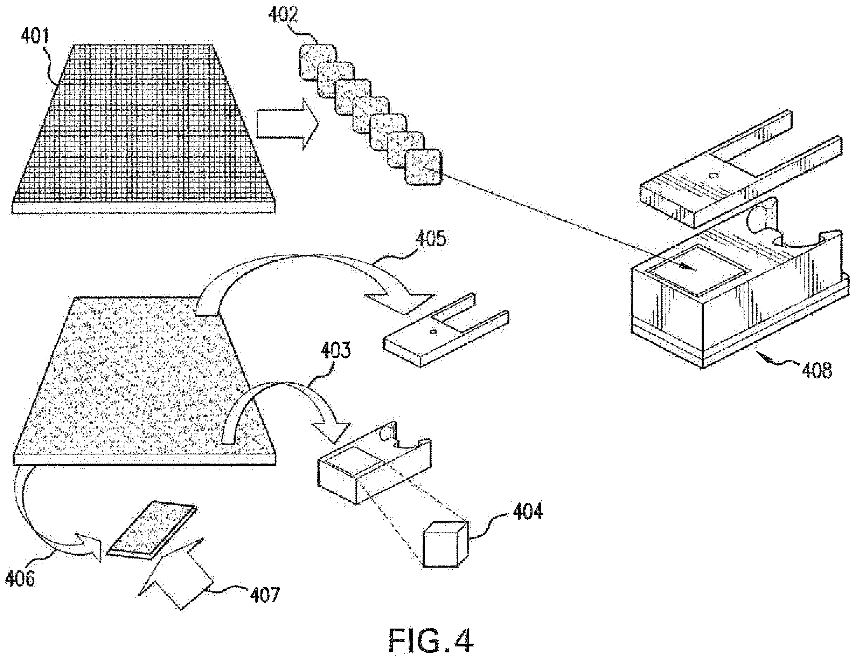

As illustrated in **Fig. 2B****,** cover plate **204** is substantially parallel to base plate **206,** and base plate **206** is substantially parallel to, and disposed on, bottom film **207.** **Fig. 2B** shows a cross-section of the sensor having a wafer-style stacking of the bottom film **207,** base plate **206,** vibratable sensor **201,** housing **202,** and cover plate **204,** wherein the layers may be hermetically sealed and the vibratable sensor **201** is disposed in the cavity **208** of the housing **202** in the illustrated embodiment. Techniques for hermetically sealing the layers of the sensor include but are not limited to diffusion bonding. In certain embodiments, bottom film **207** is sealed by controlled environment methods that minimize oxygenation and other impurities of the bottom film, where conventional, uncontrolled sealing techniques may damage the bottom layer **207.** Remaining volume within the cavity **208** may be filled with an incompressible fluid, through the fill port **205 (****Fig. 2A****)** of the cover plate **204.** After filling is complete, fill port **205** is temporarily or permanently sealed with different welding technologies such as, for example, arc, laser, resistance, ultrasonic, or torsional, or by diffusion bonding, swedging, adhesives gaskets, capillary seals, or other suitable means for sealing. The manufacturing and assembly method is detailed herein below with respect to the description of **Fig. 4****.**

The overall size of the sensor device **200** depicted in **Fig. 2****,** which is desirably extremely small compared to conventional wireless devices for measuring fluid pressure, may be 0.1 mm - 1 mm in width (*w*), 0.1 mm -1 mm in depth (*d*), and 0.1 mm - 0.75 mm in height *(h).* In one embodiment, the sensor device **200** has an equal width and depth, forming a substantially cubic structure. Generally, the overall volume of the sensor device will not exceed 0.3 cubic millimeters. For the embodiment shown in **Figs. 2A, 2B****,** housing **202** has a minimum wall thickness of **300** microns. Base plate **206** has a height, *h* of approximately 100 microns. Further, base plate **206** is relatively thin compared to the overall height of the sensor device **200** depicted in **Figs. 2A, 2B****,** which may be, for example, 100 microns (base plate **206)** compared to 500 microns (for the overall sensor device). Such a configuration provides more robustness for sensor device **200.** In addition, cavity **208** desirably has a height of approximately 400 micronsmeasured from the surface of base plate **206** abutting cavity **208** to the surface of cover plate **204** abutting cavity **208** -- but is at least 100 microns in height, and is relatively large compared to the overall height of the device, 400 microns (cavity) versus 500 microns (height of the overall sensor device) in the example of **Figs. 2A, 2B****.**

The above principles allow for an overall reduction in size from conventional wireless devices for measuring fluid pressure, because the above principles allow for a relatively thick (greater than 1 micron, for example, 2 microns) sensor membrane **209** which is accurate and robust enough to obviate further active components and/or sensor arrays.

Another aspect not forming part of the current invention relates to a method for determining pressure in the porto-hepatic venous system. Once the sensor device **100 (****Fig. 1****)** is located, data is collected using the transmitter/receiver array **103, 104, 106** as illustrated in **Fig. 1****.** High frequency **101** and low frequency **102** acoustic beams are generated by high frequency **103** and low frequency **104** transmitters, and applied to sensor device **100.** Acoustic beams **101, 102** are typically initiated by positioning the transmitters **103, 104** in close but external proximity to the sensor device **100,** where "close proximity" is any distance sufficient to apply acoustic beams **101, 102** to sensor device **100** in accordance with the devices and methods herein. Vibrations from the sensor, interrogated and excited by the high frequency **101** and low frequency 102 acoustic beams, create modulated acoustic waves **105,** due to the vibration of the vibratable sensor **201 (****Fig. 2****).** Modulated acoustic waves **105** are detected by high frequency receiver **106** which is also placed in close proximity to sensor device **100.**

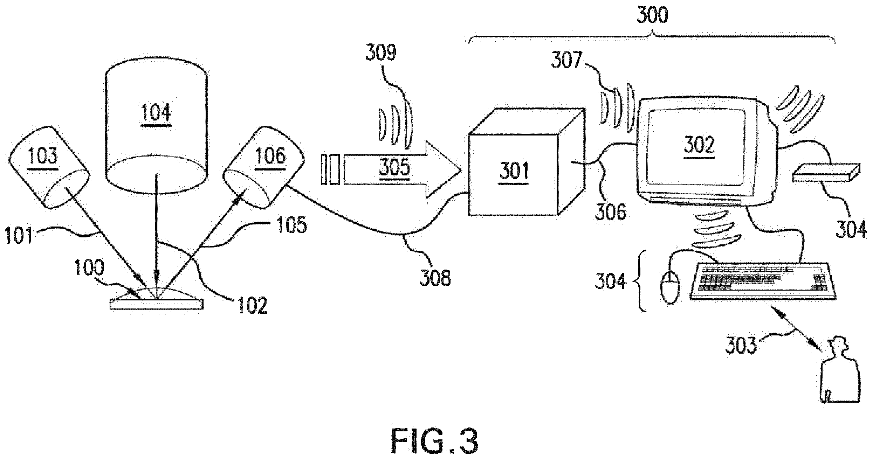

**Fig. 3** shows one embodiment of a processing and display system **300** of the system of the current invention and illustrates operation of the sensor device in the system. **Fig. 3** makes reference to **Fig. 1****,** which illustrates a generic sensor device **100** of the system of the invention, however the processing and display system **300** of **Fig. 3** applies equally to the sensor device **200** as illustrated in **Figs. 2, 2A** and **2B****.** Thus, for purposes of describing the operation of the sensor device and system with reference to **Fig. 3****,** sensor device reference numbers **100** and **200** are used interchangeably.

Referring to **Fig. 3****,** high frequency receiver **106** transmits data **305** to processing unit **301.** Data **305** may include radio waves, electrical signals, digital signals, waveform signals, or any other means sufficient for communicating the acoustic properties of modulated acoustic waves **105,** as received by high frequency receiver **106.** Processing unit **301** interprets data **305** using the properties of modulated acoustic waves **105** to determine a frequency response of the sensor device **100.** The frequency response of the sensor is defined herein as the frequency of vibrations, including at least one resonance frequency, emitted by the sensor in response to the transmission of ultrasonic vibrations from transmitters **103, 104,** at a given ambient pressure. For example, the frequency response of sensor device **100** is known when sensor device **100** is subject to "normal", *i.e.,* non-symptomatic, physiological conditions. In the portal venous system, "normal" conditions are a pressure approximately 5 mmHg or less, and a pressure gradient between the portal and the hepatic vein of approximately 10 mmHg or less. The internal pressure of sensor device **100** -- *i.e*., the pressure within cavity **208** -- is known and substantially constant. In the portal venous system, the frequency response of sensor device **100** changes in accordance with changes in the venous pressure. Low-frequency acoustic waves **102,** for example at 50 kHz, will stimulate at least one frequency response of vibrations in sensor device **100,** at a given pressure, by exciting vibrations in vibratable sensor **201 (****Fig. 2****).** High frequency acoustic waves, for example 750 kHz, may be used to interrogate the excited vibratable sensor **201 (****Fig. 2****).** This results in modulated acoustic waves **105** that can be detected by receiver **106.** High frequency acoustic waves are meant to interrogate, not to excite, the membrane **209** of the vibratable sensor **201,** and preferably minimally interact with the membrane **209** to maximize linearity of the system.

One type of frequency response which may be measured is a resonance frequency. For example, resonance frequency(-ies) of the sensor device **100** may be identified as the frequency(-ies) which exhibit peak vibration amplitudes returned from the sensor device **100.** In an alternative embodiment, the resonance frequencies are absorbed by bottom film **207,** and therefore do not materialize as vibrations generated by the sensor device **100,** and are identified as the frequencies where vibrations are not returned from the sensor device **100,** or where the minima of amplitude vibrations returned from sensor device **100** exist. The difference between the actual resonance frequency excited in the sensor device **100** and the resonance frequency of the sensor device under normal conditions is correlated to the difference in pressure between normal conditions and the actual blood pressure. Thus, actual portal venous pressure is calculated based on the measured resonance frequencies of sensor device **100.**

In one embodiment**,** the low frequency transmitter is an annular low frequency piezoelectric transducer having a working range of 0-100 kHz, 30-100 kHz, or 50-100 kHz, for example, depending on the precision required. It is, however, noted that any other suitable low frequency transducer known in the art may be used.

In another embodiment**,** the high frequency transmitter **103** is an annular high frequency transmitting transducer, implemented as a low noise (*i.e..*, low-range or low-bandwidth) frequency generator unit designed to generate a high frequency acoustic wave **101** at, for example, 750 kHz. It is noted, however, that other different values of the high frequency acoustic wave may also be used.

In one embodiment high frequency receiver **106** is a disc-like high frequency receiving piezoelectric transducer. The annular high frequency transmitter **103** and the high frequency receiver **106** are, for example, a model CLI 7900 general-purpose ultrasonic probe, commercially available from, for example, Capistrano Labs, Inc., San Clemente, Calif., USA. When the acoustic waves including the high frequency acoustic waves **101** and low frequency acoustic waves **102** are directed at the sensor device **100,** the high frequency receiver **106** receives the modulated acoustic waves **105** which are excited in the sensor device **100** as well as other noise, e.g., signals that are reflected from other materials in the measurement environment or interference. The high frequency receiver **106** generates an electrical signal representative of the returning acoustic signals that it receives. The electrical signal produced by the receiver **106** is processed by the system described herein, for example as shown in **Fig. 3****.**

In another embodiment, low frequency transmitter **104** has a working range of 30-90 kHz, and transmits acoustic frequencies, for example, at 50 kHz; high frequency transmitter **103** transmits, for example, at approximately 750 kHz with a narrow bandwidth (range); high frequency receiver **106,** under the example, operates in the range of 750 (high) ± 50 (low) kHz. Low frequency transmitter **104,** high frequency transmitter **103,** and high frequency receiver **106** may alternatively operate in any range suitable for use with the devices and methods disclosed herein, and as particularly required for measuring fluid pressure in particular environments.

High frequency receiver **106** is also a transducer, and is used for receiving the signals returning from the sensor when the sensor is interrogated by the high frequency acoustic waves **101.** For example, the transducer may be implemented using suitable piezoelectric transducers, but any other type of transducers known in the art may be used to implement the transducers, such as, but not limited to, capacitive transducers, wideband capacitive transducers, composite piezoelectric transducers, electromagnetic transducers, various transducer array types, cMUTs, cymbal transducers and various suitable combinations of such transducers configured for obtaining different frequencies and/or beam shapes. For example, acoustic receivers manufactured by Vemco, PCB Piezoelectronics, and Hardy Instruments may be used.

Modulated acoustic waves **105** are the result of combining high frequency acoustic waves **101** and low frequency acoustic waves **102** in a reversible manner, in order to achieve a waveform with a desired frequency, wavelength, and/or amplitude. Unmodulated noise, for example caused by reflections of acoustic waves off of materials in the sensor device **100** environment, is thus distinguished from the modulated acoustic waves **105** that are excited by the sensor device **100.** When the received signal amplitude (in dB) is analyzed according to the frequency (in MHz), the amplitude peaks at the resonance frequency of the sensor device **100.** High frequency receiver **106** communicates the modulated acoustic waves **105** to a processing and display system, detailed in **Fig. 3****,** for interpretation and use.

In one embodiment, vibrations excited in sensor device **100** are distinguished from noise by correlating pressure measurements to a heart rate or pulse measurement. In this embodiment, a plurality of pressure measurements are taken during the interrogation period, for example, at least one cycle of expansion and contraction of the heart (pulse cycle). During the pulse cycle, the pressure of the entire vascular system will change continuously as the heart draws blood in and forces blood out. Accordingly, an acoustic signal that changes in a consistent manner correlated to the pulse cycle demonstrates an excitation in the sensor. Noise reflected from, for example, surrounding tissues in the interrogation environment, does not produce such a continuously changing signal that is correlated to the pulse cycle. The above features are not limited to a single embodiment; rather, those features and functions may be applied in place of or in conjunction with the other embodiments and concepts herein. The pulse cycle and waveform may be measured by an external device, for example using a pulse oximeter, heart rate monitor, ECG, etc. Optionally, such instruments may be connected to the pressure monitoring system of the invention to input the pulse or pulse waveform into the system for correlation with the acquired pressure waveform from the sensor to determine the validity of the acquired signal.

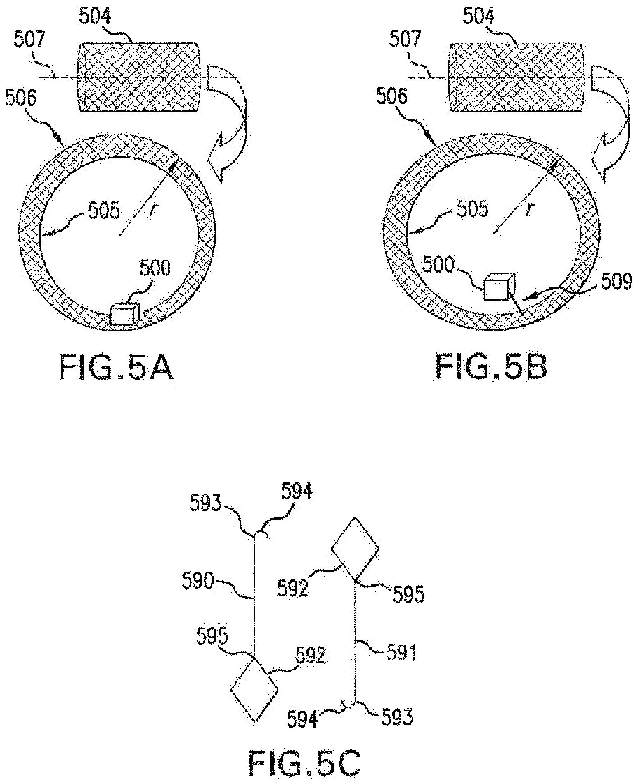

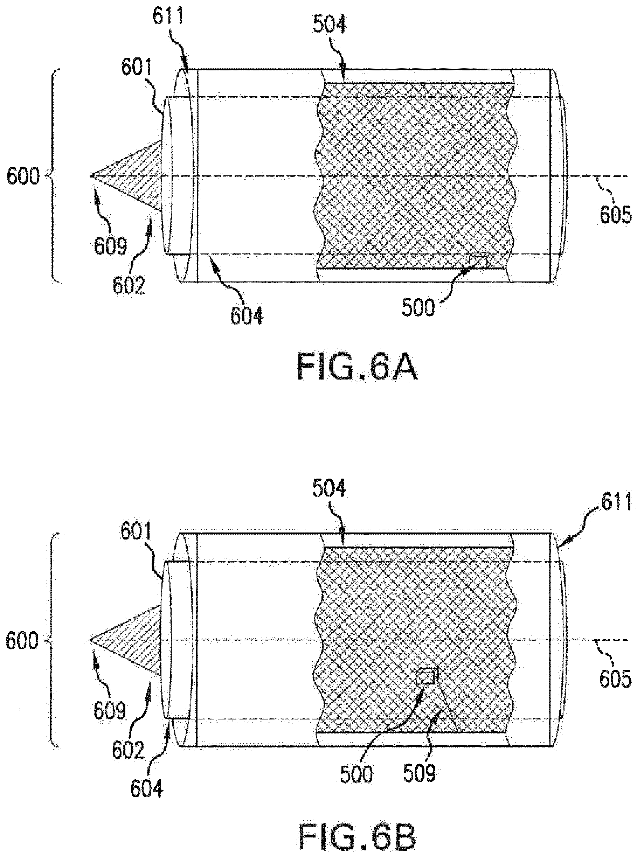

In operation, sensor device **100** is disposed in a measurement environment, for example, implanted in an area, vessel, artery, or the like, where pressure measurements are desired. The sensor system may be implanted by methods including, for example, portal venous catheterization procedures to position the sensor device **500** in the portal vein shown, for example, via scaffoldings **504** illustrated in **Figs. 5-6****.** In such a procedure a percutaneous transhepatic approach to the portal vein may be employed, for example inserting the cannula **601** into a subject between the ribs and puncturing through to the portal vein. For the hepatic vein, the sensor device **500** may be inserted, for example, by transjugular hepatic vein access, similar to the procedure used in hepatic vein pressure-gradient measurements. In this procedure, a catheter is inserted into the jugular vein in the neck and advanced into the hepatic vein via the vena cava. The portal vein is also accessible by puncture from the hepatic vein, after a catheter has been inserted via transjugular hepatic procedures similar to the implantation of transjugular intrahepatic portosystemic shunts. Implantation into the portal vein may also involve traversing a TIPS shunt, in which case the patency of the TIPS shunt may be non-invasively monitored. Implantation is typically performed by an interventional radiologist under fluoroscopic guidance. Sensor device 500 is guided to the intended position using catheter delivery system **600,** for example, as shown in **Figs. 6A-6B****.** Once deployed in the intended location, sensor device **500** remains in the vessel or area. Other methods for deploying the sensor as are known in the art may alternatively be employed. Non-limiting examples of such deployment methods include, but are not limited to, those described in U.S. Patent No. 6,331,163 to Kaplan and U.S. Patent Publication No. 2005-0124896 to Richter.

According to one aspect not forming part of the current invention, the implanted sensor device **100** is subjected to both high and low frequency acoustic waves **101, 102,** the latter excites vibrations in the sensor device **100,** and the reflected high frequency acoustic waves are then manifested as modulated acoustic waves **105.** High frequency receiver **106** receives the modulated acoustic waves **105** and communicates the properties of the modulated acoustic waves **105** to a processing and display system, detailed in **Fig. 3****,** for interpretation and use.

Returning to **Fig. 3** which shows one embodiment of a processing and display system **300** of the current invention, data **305** from high frequency receiver **106** is transmitted to a processing unit **301** which determines the pressure of the environment surrounding the sensor device **100.** Data **305** is communicated between high frequency receiver **106** and processing unit **301** via a wired **308** or wireless **309** connection. Wired connection **308** is, for example, an electronic cable or integral connection, or the like. Wireless connection **309,** for example, operates by transmitting radio waves, acoustic waves, or other known media for remotely communicating data.

Processing unit **301** may comprise a computer, workstation, or other electrical or mechanical device programmed to perform the data conversions and/or displays described herein and as needed for the method of use. By way of a non-limiting example, the disclosure may be practiced on a standard workstation personal computer, for example those manufactured by Dell, IBM, Hewlett-Packard, or the like, and which typically include at least one processor, for example those manufactured by Intel, AMD, Texas Instruments, or the like. Processing unit **301** also comprises dedicated hardware and/or software, *e.g*., a data capture system such as the National Instruments PCI-6115 data capture board or may be comprised of a custom designed device for that purpose.

The output of processing unit **301** is a pressure measurement that is converted to a usable, displayable measurement either by processing unit **301** or display unit **302,** or a combination thereof. For example, pressure measurements may be reported in numerical units of mmHg or Torr or maybe displayed with relation to a predefined arbitrary scale. Display unit **302** may comprise a monitor, numerical display, LCD, or other audio or visual device capable of displaying a numerical measurement. As shown in the embodiment of **Fig. 3****,** display unit **302** is connected to or integral with processing unit **301** by connection **306,** for example in the case of a computer with processing and display units, which optionally includes as a remote element, separate wired element, or integral element to processing **301** and/or display **302** units, interface **303** and input/output elements **304,** such as a keyboard, mouse, disk drive, optical pen, or the like, to allow a user to collect, manipulate, track, and record data. Connection **306** may optionally be a remote connection **307,** operating by transmission of radio waves, acoustic waves, or other known remote transmission methods.

One aspect not forming part of the current invention is directed to a method of monitoring PHT. The sensor device **100** may be implanted in either or both of the portal and/or hepatic veins according to the procedures described herein or known. Once implanted in the porto-hepatic venous system, the method comprises the steps of: subjecting the sensor device **100** to ultrasonic vibrations from high frequency **103** and low frequency **104** transmitters; receiving the frequency response of one (or each) of the sensor devices **100;** determining a resonance frequency of the (or each) sensor device **100** from the received frequency response; determining ambient fluid pressure surrounding the (or each) sensor device **100** from the resonance frequency of the (or each) sensor device **100;** determining a pressure gradient between each sensor device **100** (in each of the portal and hepatic veins) wherein an elevated gradient (generally greater than 10 mm Hg) is indicative of an active portal hypertension condition in need of treatment; and displaying and/or recording the pressure measurements according to the system described with respect to **Fig. 3****.** Thus, the pressure of the portal and/or hepatic veins may be independently interrogated, determined, and displayed. Where the pressure gradient between the portal and hepatic veins is desired, one sensor may be implanted in each of those systems, and data captured for each sensor in the manner described above. The numerical measurement of the hepatic vein pressure, for example, could then be subtracted by further processing from the numerical measurement of the portal vein pressure, providing the gradient, or difference in pressure, between the two systems.

The method of monitoring a pressure gradient between the portal and hepatic veins includes the additional step of delineating between each sensor while performing the interrogation. The mechanism for the differentiation can be one of the following or both: (i) differences in frequency responses between the sensors may be detected by changing the dimensions of the membrane while maintaining the pressure ranges and accuracy of the sensor (*i.e.,* one sensor will have a frequency response at a defined pressure between 30-50 kHz while the other may have a frequency response of 60-80 kHz at the defined pressure). Such a design entails a low frequency transmitter with a wide enough bandwidth to enable the operation of both sensors (*i.e.,* between 30-50 and 60-80 kHz), or two or more low-frequency transmitters, one for each type of sensor; (ii) a narrow high or low (or both) frequency acoustic field is applied to the vicinity of the sensors to precisely locate each sensor during interrogation while acoustically isolating any other sensors in the vicinity.

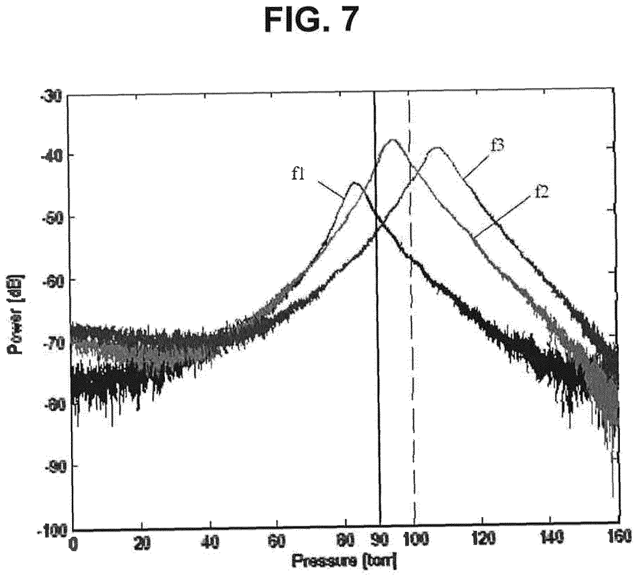

In one embodiment, determining the pressure in the portal and/or hepatic veins comprises obtaining the mean pressure by a phase inversion method of calculation, which relies on small pressure oscillations created by the heartbeat. The small pressure oscillations exist around the mean pressure value which is to be measured. In order to determine the mean pressure value to be measured, a receiver as described for example with respect to **Fig. 3** measures the response power of the sensor device, which is the amplitude of the oscillation of the vibratable sensor and is measured in decibels (dB). As illustrated in **Fig. 7****,** the small pressure oscillations occur around a particular mean value - for example 90 Torr, indicated by the solid vertical line. When the sensor device is excited by certain frequencies, for example f1 and f2, the response power is an increasing function of the pressure, whereas excitation by another frequency, f3, results in a response power that is a decreasing function of the pressure. As a direct result the response power of f1 and f2 oscillate in phase with each other (and with the pressure) and that of f3 oscillates with an opposite phase. When the small pressure oscillations occur around a different mean value - for example 100 torr, indicated in **Fig. 7** by the dashed vertical line - the response power of f1 is an increasing function of the pressure, whereas that of f2 and f3 are decreasing functions of the pressure. As a result, the response power of f1 oscillates in phase with the pressure, and that of f2 and f3 oscillated with an opposite phase. The phase inversion algorithm is based on these observations. The resonance frequency of the sensor device at the mean ambient pressure is that around which the phase inversion occurs. In this embodiment, the pulse cycle and waveform may be measured with an external device for correlation with the acquired pressure waveform from the sensor.

This technique is particularly applicable to PHT since only a mean pressure reading is necessary.

With reference now to **Fig. 4****,** one example of a manufacturing method is shown for a sensor device in accordance with the devices and methods described but not claimed herein. In step **401,** vibratable sensors are etched and cut from a panel of material to produce a plurality of individual vibratable sensors **402,** each of which is hermetically sealed with a bonding layer **211** (illustrated in **Figs. 2****,** **2B****)** made of, for example, Pyrex^{®}, which may be anodically bonded to one side of vibratable sensor **402,** or attached by brazing, welding (such as, for example, arc, laser, resistance, ultrasonic, or torsional), diffusion bonding, vapor deposition, adhesives, epoxies, or the like. Each vibratable sensor may then be assembled into a sensor device directly or may be further processed to be inserted into a housing cavity, as described below. Housing defining a cavity may be created in parallel steps, in which an individual housing is etched and cut **403** from larger panels of material and assembled **404** into a housing having a cavity. Cutting is accomplished by any suitable method, *e.g.*, chemical etching, laser cutting, mechanical cutting, plasma cutting, punching, or the like. In a similar fashion if a cover plate is desired, a cover plate and fill port are machined **405** from a larger panel of material. Similarly, a base plate may be machined **406** from a larger panel of material. In one embodiment, a bottom film is hermetically sealed to the face of the base plate opposite the face that will abut the stacked assembly, in step **407,** via brazing, welding (such as, for example, arc, laser, resistance, ultrasonic, or torsional), diffusion bonding, vapor deposition, adhesives, epoxies, or the like. In another embodiment, the bottom film is not used. A vibratable sensor is then inserted into the cavity in the housing and the sensor-housing assembly is disposed on a base plate in a wafer-style stacking arrangement **408** (see also **Fig. 2B**). As part of step **408,** the cover plate is disposed on the housing and encloses the vibratable sensor in the cavity, and the base plate and housing, and housing and cover plate, are hermetically sealed via brazing, welding (such as, for example, arc, laser, resistance, ultrasonic, or torsional), diffusion bonding, vapor deposition, adhesives, epoxies, or the like. In a further, non-illustrated step, the empty space of the cavity surrounding the vibratable sensor is filled with an incompressible fluid via the fill port in the cover plate, and the fill port is subsequently hermetically sealed using brazing, welding (such as, for example, arc, laser, resistance, ultrasonic, or torsional), diffusion bonding, or the like.

In the embodiment where the sensor without a housing is desired, the sensor is further manufactured by attaching the vibratable sensor to an anchoring means. In one embodiment, a bonding layer (illustrated as **211** in **Figs. 2, 2B**) is attached to the vibratable sensor by brazing, welding, diffusion bonding, vapor deposition, adhesives, epoxies, or the like. The bonding layer provides a surface to attach the sensor to a support structure, for example an anchoring means. The bonding layer and support structure may be joined by brazing, welding, diffusion bonding, vapor deposition, adhesives, epoxies, or the like. In one embodiment, the bonding layer comprises Pyrex^{®}.

The sensor device with or without a housing may be fixed to a desired support structure by various means known in the art. A support structure such as, for example, an annular shaped structure may be pressed against the vessel wall wherein the sensor device is attached thereto. In another embodiment, hooks, tethers, or other fixation devices may be used to fix the sensor into the desired position. **Fig. 5** shows attachment of sensor device **500** to an exemplary anchoring means; in this example, sensor device **500** may be diffusion bonded, welded, brazed, soldered, or otherwise suitably attached to an inner side **505** of scaffold **504.** Scaffold **504** may be a stent-like structure, which is a tubular device that is typically implanted in a damaged vessel or artery to maintain the opening of the vessel or artery, as described for example in U.S. patent no. 7,763,064 to Pinchasik. Scaffold **504** comprises inner side **505,** an outer side **506,** and a longitudinal axis **507.** In some embodiments, scaffold **504** has a high degree of radial force in direction *r*, in order to hold a vessel or artery open. When a stent is used as scaffold **504** it is preferred that the stent provide sufficient radial resistance in direction *r*(*see* **Fig. 5B**) to hold the stent in a constant position in the vessel; *i.e.,* to secure the sensor in the desired position. U.S. patent no. 7,763,064 to Pinchasik describes such scaffolds.

The scaffold **504** may be either self-expanding or expanded by an inflatable balloon. In one embodiment the scaffold is balloon expandable, and the delivery system includes an inflation lumen. An inflation balloon may be coaxially disposed on the outside of the cannula or catheter. Scaffold **504,** including passive sensor **500,** is crimped onto the inflation balloon for insertion and placement. After scaffold **504** is in place within the body, inflation balloon is inflated under the control of the operator. Scaffold **504** expands until it reaches a desired diameter within a vessel or area. The inflation balloon is then deflated and removed, leaving scaffold **504,** including sensor device **500,** within the vessel or area. Scaffold **504** comprises, for example, nitinol, stainless steel, cobalt chromium, or other biocompatible materials with sufficient elasticity and plasticity to expand under the force of inflation balloon and remain securely in place after expansion.

In another embodiment, scaffold **504** is made from Nitinol, or another self-expandable material that will expand, for example, under higher, *in vivo,* temperatures and pressures. For certain sensor devices, it may be desirable to deploy the sensor without the need for an inflation balloon to prevent damage to the attached sensor device. U.S. 2006/0122691 to Richter, for example, discusses such materials and their use in scaffolds.

Scaffold **504** comprises, for example, nitinol, stainless steel, cobalt chromium, or other biocompatible materials with sufficient elasticity and plasticity to expand under the force of inflation balloon inflating and remain securely in place after expansion. Typically, an animal body will respond to the presence of a foreign object, such as the scaffold **504,** by forming neointima, which aids in securing the scaffold **504.** U.S. patent publication no. 2006/0122691 to Richter, for example, discusses neointimal growth and securing scaffolds in place by burying the scaffold in neointima.

**Fig. 5B** shows an embodiment where sensor device **500** is tethered to scaffold **504** via a lead line **509,** which is a stent strut, cable, wire, or other suitable material that is capable of resisting the force of blood-flow and potential influence on the position of the device, and is bioinert as herein discussed. A lead line is attached to sensor device **500** and scaffold **504** by welding, brazing, tying, adhesives, or the like, or may be an integral part of scaffold **504.**

An alternative method of implanting a sensor device of the invention in a measurement environment involves the use of an anchoring mechanism other than a scaffold. **Fig. 5C** illustrates an embodiment for an anchoring mechanism from the prior art comprising a first support leg **590** and a second support leg **591** which are attached at a first end **595** to the sensor housing **592** of a sensor device of the invention. At a second end **593,** each support leg **590, 591** has a protrusion **594** in the shape of a hook, or the like. The protrusions **594** of the anchoring mechanisms attach to the tissues or walls of vessels in which the sensor housing **592** is implanted, thereby securing the assembly.

The sensor of the invention may be delivered to the target site by various methods known in the art. Implantation into the portal vein may be done via a transhepatic puncture using either an intracostal or subxiphoid approach. Implantation may also be done using a transjugular approach that would necessitate an intrahepatic puncture from the hepatic to portal venous systems. **Fig. 6** shows one embodiment of a delivery system **600** for use in delivering the sensor device **500** and attachment means to the sensing environment. As illustrated in **Fig. 6****,** the delivery system **600** comprises an intravenous cannula or catheter that includes an internal tube **604** having a lumen about a longitudinal axis **605** and an external or outer tube **611.** A cut-away view of **611** in **Figs. 6A** and **6B** shows the scaffold **504** with the sensor **500** may be coaxially disposed about the internal tubular structure **604** of the delivery system, for example a cannula or catheter. In this embodiment, the scaffold **504** is self-expanding. As shown in **Fig. 6A** and **6B****,** the scaffold **504** may be crimped around the internal tube **604** and held in the compressed delivery configuration by the outer tube **611.** To deploy the scaffold **504,** the outer tube **611** is removed to permit the scaffold **504** to expand and engage the vessel lumen. Once expanded the interior tube **604** may be withdrawn leaving the scaffold **504** in the vessel, with the sensor **500** exposed to the ambient fluid of the vessel. In the embodiment illustrated in **Fig. 6****,** the cannula **604** or catheter has at a distal end **601** a trocar **602** having a sharp tip **609** for puncturing the bodily tissues and organs is coaxially disposed inside the lumen of the cannula **601.** Alternatively the cannula **604** or catheter on which the scaffold **504** is disposed may be threaded through a needle-based system, which is used to penetrate the tissue and into the appropriate vessel, and advanced to the location where the sensor device **500** is to be deployed. Preferably, the tip of the catheter has a soft, rounded tip.

**Fig. 6A** shows an embodiment, wherein the scaffold **504** and sensor device **500** depicted in **Fig. 5A** are mounted on the catheter delivery system **600** coaxially. **Fig. 6B** shows a similar delivery system for a sensor **500** attached to scaffold **504** by lead line **509.** When the scaffold **504** is implanted and expanded, sensor **500** is engulfed by the bloodstream, for example.

Once in place, the sensor may be located by various methods known in the art. For example, the presence and the intensity of Doppler shifted sideband peaks in the frequency response of the sensor may be used to identify or locate the sensor in the body and to assist the centering of the interrogating ultrasound beam on the sensor(s). The sensor reflects the carrier frequency ultrasound signal (with Doppler shift) with much higher amplitude than any tissue in the human body, thus the identification and localization of the sensor and the centering of the interrogating beam may be performed by searching for a significant Doppler effect in the received signal. If the interrogating beam is scanned across the region in which the sensor is implanted or located, the beam is centered on the sensor when the sideband frequency's amplitude is maximal. When correlating a received signal to a pulse cycle measurement, the pulsatile pressure changes the signal amplitude of the Doppler sideband frequency (or frequencies) during the pulse cycle time. These pulsatile pressure induced sideband amplitude changes are present only in the signal reflected from the vibratable membranes of the sensor. Maximizing the amplitude of these pulsatile (periodic) amplitude changes may also be used by the system for sensor identification and for beam centering. Thus, the operator or user of the device may scan the interrogating beam in the region where the implanted sensor is assumed to be positioned and look for the presence of a sideband component (or components) at the expected frequency (or frequencies) having an amplitude which periodically varies in time at a rate similar to the blood pulse rate. In accordance with an embodiment of the invention, the pulsating sideband component may be visually detected on a display device coupled to the system. The interrogating beam may then be centered by carefully changing the beam direction and/or orientation in until the amplitude of the amplitude of the periodically varying sideband is maximal.

The system's operator may then carefully scan the interrogating beam position for fine-tuning the best beam position. The beam's position may be fine-tuned or optimized by slowly changing the beam direction and/or orientation until the amplitude of the sideband peak(s) is the maximized. By maximizing the sideband amplitude the operator may ensure a good signal to noise ratio by maximizing the received energy at the sideband frequency or frequencies. Maximizing the amplitude of sideband frequency (or frequencies) may also contribute to improving the signal-to-noise ratio and therefore the measurement accuracy and/or the inter-test and/or intra-test accuracy, repeatability and sensitivity. After beam centering, the operator may use the system for determining the blood pressure by determining the resonance frequency of the sensor(s) as disclosed in detail herein and computing the blood pressure from the determined resonance frequency (or frequencies).

## Claims

1. A vibratable sensor (201) for measuring fluid pressure, the sensor (201) comprising:
a sensor membrane (209) and a chamber (210);
wherein the sensor membrane (209) has a frequency response to ambient pressure conditions and has a thickness in the range of 1 micron - 200 microns and forms one side of the chamber (210) of the vibratable sensor 201, and
the chamber (210) being defined by the sensor membrane (209) and a plurality of walls (203) and the chamber (210) being sealed with a bonding layer (211), forming a side of the chamber (210) opposite the sensor membrane (209), the chamber (210) being sealed with a compressible gas of predefined pressure disposed therein;
wherein the volume of the vibratable sensor does not exceed a maximum volume of about0.3 cubic millimeters.

2. The sensor (201) according to claim 1, wherein the bonding layer (211) comprises a substance selected from glass and silicon.

3. The sensor (201) according to claim 1 or 2, wherein the walls (203) of the chamber (201) are substantially perpendicular to the membrane (209).

4. The sensor (201) according to any one of claims 1 to 3, wherein the sensor membrane (209) has a thickness selected from the group consisting of 1 micron, 1.5 microns, 2 microns, 2.5 microns, and 5 microns.

5. The sensor (201) according to any one of claims 1 to 4, wherein the sensor (201) measures 0.3 mm x 0.5 mm x 0.5 mm.

6. The sensor (201) according to any one of claims 1 to 5, wherein the sensor (201) has a volume in cubic millimeters selected from the group consisting of 0.005-0.008, 0.01-0.09, and 0.1-0.3.

## Patentansprüche

1. Schwingungsfähiger Sensor (201) zum Messen eines Fluiddrucks, wobei der Sensor (201) aufweist:
eine Sensormembran (209) und eine Kammer (210);
wobei die Sensormembran (209) auf Umgebungsdruckbedingungen mit einem Frequenzgang reagiert, eine Dicke in dem Bereich von 1 Mikrometer bis 200 Mikrometer aufweist und eine Seite der Kammer (210) des schwingungsfähigen Sensors (201) bildet, und
die Kammer (210) durch die Sensormembran (209) und eine Vielzahl von Wänden (203) begrenzt ist und die Kammer (210) mit einer Verbindungsschicht (211) abgedichtet ist, die eine der Sensormembran (209) gegenüberliegende Seite der Kammer (210) bildet, wobei die Kammer (210) mit einem darin befindlichen komprimierbaren Gas eines vordefiniertem Drucks abgedichtet ist;
wobei das Volumen des schwingungsfähigen Sensors ein Maximalvolumen von etwa 0,3 Kubikmillimetern nicht überschreitet.

2. Sensor (201) nach Anspruch 1, wobei die Verbindungsschicht (211) eine Substanz aufweist, die aus Glas und Silizium ausgewählt ist.

3. Sensor (201) nach Anspruch 1 oder 2, wobei die Wände (203) der Kammer (201) im Wesentlichen senkrecht zu der Membran (209) sind.

4. Sensor (201) nach einem der Ansprüche 1 bis 3, wobei die Sensormembran (209) eine Dicke aufweist, die aus der Gruppe bestehend aus 1 Mikrometer, 1,5 Mikrometer, 2 Mikrometer, 2,5 Mikrometer und 5 Mikrometer ausgewählt ist.

5. Sensor (201) nach einem der Ansprüche 1 bis 4, wobei der Sensor (201) 0,3 mm × 0,5 mm × 0,5 mm misst.

6. Sensor (201) nach einem der Ansprüche 1 bis 5, wobei der Sensor (201) ein Volumen in Kubikmillimetern aufweist, das aus der Gruppe bestehend aus 0,005-0,008, 0,01-0,09 und 0,1-0,3 ausgewählt ist.

## Revendications

1. Un capteur pouvant vibrer (201) pour mesurer la pression d'un fluide, le capteur (201) comprenant :
une membrane de capteur (209) et une chambre (210) ;
dans lequel la membrane de capteur (209) présente une réponse en fréquence aux conditions de pression ambiante, a une épaisseur de 1 micron à 200 microns et forme l'une des parois de la chambre (210) du capteur vibrant (201), et
la chambre (210) est délimitée par la membrane de capteur (209) et d'une pluralité de parois (203), et la chambre (210) étant scellée par une couche de liaison (211) formant une face de la chambre (210) opposée à la membrane de capteur (209), la chambre (210) étant scellée avec un gaz compressible à une pression prédéfinie disposé à l'intérieur ;
dans lequel le volume du capteur vibrant ne dépasse pas un volume maximal d'environ 0,3 millimètre cube.

2. Le capteur (201) selon la revendication 1, dans lequel la couche de liaison (211) comprend une substance choisie parmi le verre et le silicium.

3. Le capteur (201) selon la revendication 1 ou 2, dans lequel les parois (203) de la chambre (201) sont sensiblement perpendiculaires à la membrane (209).

4. Le capteur (201) selon l'une quelconque des revendications 1 à 3, dans lequel la membrane du capteur (209) présente une épaisseur choisie parmi le groupe constitué de 1 micron, 1,5 micron, 2 microns, 2,5 microns et 5 microns.

5. Le capteur (201) selon l'une quelconque des revendications 1 à 4, dans lequel le capteur (201) présente des dimensions de 0,3 mm × 0,5 mm × 0,5 mm.

6. Le capteur (201) selon l'une quelconque des revendications 1 à 5, dans lequel le capteur (201) présente un volume en millimètres cubes choisi parmi le groupe constitué de 0,005-0,008, 0,01-0,09 et 0,1-0,3.
